# EUROPEAN PATENT SPECIFICATION

(11) **EP 0 742 435 B1**
(45) Date of publication and mention of the grant of the patent: **23.01.2002**
(21) Application number: 96400962.5
(22) Date of filing: 06.05.1996
(51) Int. Cl.: G01N 33/48, B01F 11/00, G01N 33/49

(54) **Mixing apparatus**
Mischgerät
Appareil de mélange

(30) Priority: 09.05.1995 JP 11096595
(43) Date of publication of application: 13.11.1996
(73) Proprietor: Sysmex Corporation, Hyogo (JP)
(72) Inventor: Nakagawa, Masayuki, Harima-cho, Kako-gun, Hyogo (JP); Inoue, Hisaaki, Himeji-shi, Hyogo (JP)
(74) Representative: Jacquard, Philippe Jean-Luc

(56) References cited:
- US-A- 4 943 164

## Description

### BACKGROUND OF THE INVENTION

### 1. Field of the Invention

The present invention relates to a mixing apparatus for mixing a liquid in a vessel. More particularly, it relates to a mixing apparatus utilized for well mixing of a liquid sample and a liquid reagent contained in a vessel in an automatic analyzer.

### 2. Description of the Related Arts

Conventionally, an automatic analyzer such as a blood coagulation measuring apparatus for measuring the coagulability of blood involves mixing of a subject liquid sample (plasma) and a liquid reagent in a vessel.

Known apparatus for mixing a liquid in a vessel are, for example, one which allows the liquid to be discharged into the vessel and makes use of the discharging pressure for mixing (Apparatus A), one which allows a bar-like member to be inserted into the vessel containing the liquid and utilizes the bar-like member for mixing (Apparatus B), and one which allows an eccentric rotational movement of the vessel containing the liquid for mixing (Apparatus C) as disclosed in Japanese Examined Utility Model Publication No. Hei. 5(1993)-9069.

Apparatus A is liable to cause mixing nonuniformity because it utilizes discharging pressure. Apparatus B necessitates washing of the bar-like member every time the mixing is conducted and, moreover, when the washing is insufficient, it may possibly cause mutual contamination of liquids. Apparatus C has a drawback that it tends to be large and mechanically complicated. Moreover, there is a problem of control that the driving source such as the motor must be stopped at a predetermined position to place the vessel at a prescribed location after the mixing.

### SUMMARY OF THE INVENTION

The present invention has been made in view of the above circumstances, and the purpose thereof is to provide a mixing apparatus which can prevent the possibility of generating mixing nonuniformity or contamination, employs a small and simple mechanism, and is advantageous with respect to costs.

Accordingly, the present invention provides a mixing apparatus comprising: a hand member for holding a vessel for containing a liquid to be mixed; a support member for supporting the hand member; a mobile member which moves in a predetermined region; a vibration motor mounted to the support member; and a linking member for linking the support member to the mobile member so that the support member is conically revolvable with respect to the mobile member when the vibration motor is driven, thereby enabling conical revolution movement of the vessel held by the hand member.

### BRIEF DESCRIPTION OF THE DRAWINGS

Fig. 1 is a side view of a mixing apparatus according to one embodiment of the present invention.

Fig. 2 is a plan view of the mixing apparatus in Fig. 1.

Fig. 3 is an enlarged side sectional view of a part (around a linking member) of the mixing apparatus in Fig. 1.

Fig. 4 is an explanatory view for explaining the mixing operation of the mixing apparatus in Fig. 1.

### DETAILED DESCRIPTION OF THE PREFERRED EMBODIMENTS

As the vessels for containing a liquid to be mixed, suitable ones having various sizes, shapes and materials are selectively used. An example thereof is what is known as a cuvette made of plastic or glass which can contain 50 to 300 µl of a liquid to be measured such as a sample or a reagent.

The hand member is constructed to be able to hold or release the upper, the middle or the like portion of the vessel located at a prescribed position of a rack, a turntable or the like, depending on the needs. The hand member to be used is, for example, one which comprises a pair of right and left board-like members and a coil spring stretched over the end portions of the board-like members in such a manner that the pair of board-like members are pivoted at each of the base end portions to be horizontally rotatable by an urging power of the coil spring.

The holding portions of these board-like members are adapted to have a suitable shape and structure in accordance with the external shape, the material or the like of the vessel to be held. For example, if the vessel has a cylindrical shape, the holding portions of the board-like members have a recessed portion in accordance with the cylindrical shape of the vessel so as to hold the vessel firmly in the recessed portion, or are formed of a resilient material.

The method for holding a vessel by the hand member employs gripping and holding, in a sandwich-like configuration, of an external upper surface of the vessel in a state in which the liquid may not spill out of the vessel when the mixing apparatus is in operation, for example, in a state in which the vessel is substantially vertical.

Generally, the hand member is constructed to hold one vessel. Alternatively, however, the hand member may be constructed to hold a plurality of vessels in a row in accordance with the needs. By using such a hand member, it is possible to mix liquids in a plurality of vessels simultaneously.

The holding and releasing of the hand member are actuated by, for example, a forward and backward movement of the hand member based on the horizontal back and forth movement of the mobile member linked to the hand member via the support member and the linking member.

The support member supports the hand member. If the hand member comprises the above-mentioned board-like members and the coil spring, the support member may be, for example, a vertical board-like member supporting the base end portion of each of the board-like members for pivoting movement.

The mobile member is linked to the support member via the linking member and moves in a predetermined region. The predetermined region herein referred to represents a determined region of movement selected from a linear region (one-dimensional region), a planar region (two-dimensional region), a spatial region (three-dimensional region).

The vibration motor is mounted to the support member and generates an eccentric rotational movement. An example of the vibration motor to be used is a small-sized DC coreless motor. Preferably, the rotation speed of the vibration motor is adjusted to be in the range of, for example, 5000 to 10000rpm. More preferably, the rotation speed is in the range of, for example, 7000 to 8000rpm.

The linking member links the support member and the mobile member into a semi-fixed state. The linking connection is adapted in such a manner that the support member is capable of conical revolution movement with respect to the mobile member when the vibration motor is driven. The conical revolution movement herein referred to represents a movement wherein, when the support member revolves with respect to a point serving as a fulcrum, the support member describes a trajectory of a conical shape with its apex at the above-mentioned fulcrum and with its base having a circular shape. This movement of the support member enables the vessel held by the hand member to eccentrically revolve to rotate and mix the liquid in the vessel. The radius and the speed of this revolution movement are determined by the eccentricity and the rotation speed of the vibration motor, the position of the above fulcrum, and others.

If one wishes to attach importance to obtaining a mixing apparatus which employs a small and simple mechanism and is advantageous with respect to costs, it is more preferable that the hand member is mounted on one side of the support member and the vibration motor is mounted on the other side of the support member. In such a case, the support member revolves with a fulcrum at the linking portion linking the support member to the mobile member.

In order that the above revolution movement is well generated in a simple construction, it is preferable that the linking member comprises a resilient member and that the support member is linked to the mobile member via the resilient member. In other words, the resilient member transmits and amplifies the eccentric rotational movement of the vibration motor and changes the movement into a conical revolution movement of the support member. As the resilient member, a urethane rubber, a foamed sponge, or a coil spring may be preferably used. The Shore hardness (JIS) of the urethane rubber to be used is preferably in the range of 20 to 50 degrees, more preferably in the range of 30 to 40 degrees when a liquid of about 50 to 300 µl is to be mixed.

In view of generating the above revolution movement more effectively and smoothly, the vibration motor preferably comprises a motor body and a weight, the weight being eccentrically mounted to the motor shaft of the motor body and having a cross section of a generally semicircular shape.

The present invention will be hereinafter detailed, in conjunction with the attached drawings, by way of an embodiment thereof, which is not to be construed as being intended to limit the scope of the present invention.

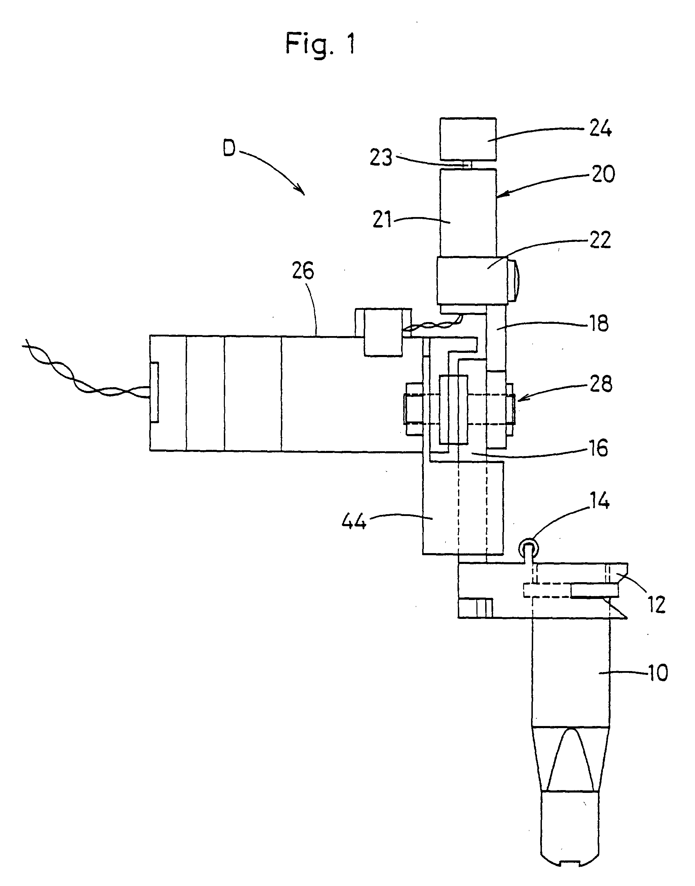

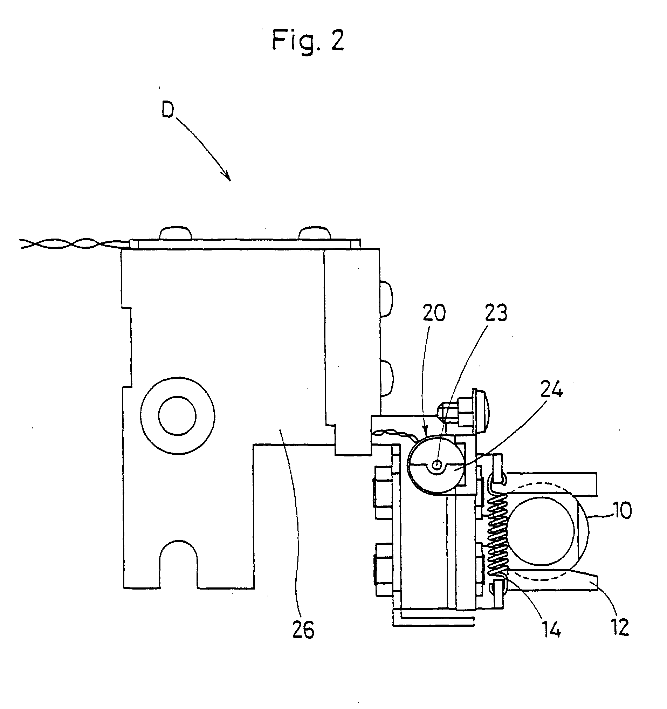

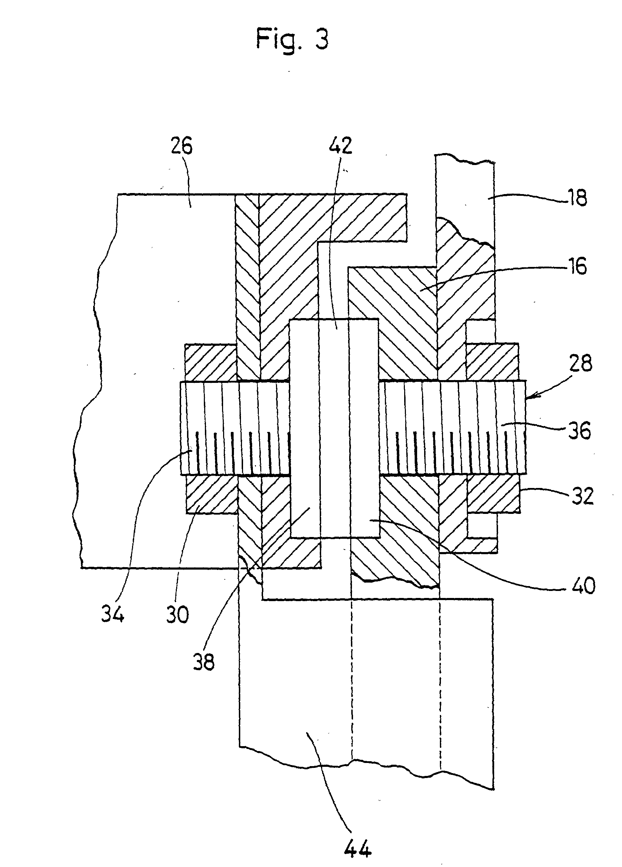

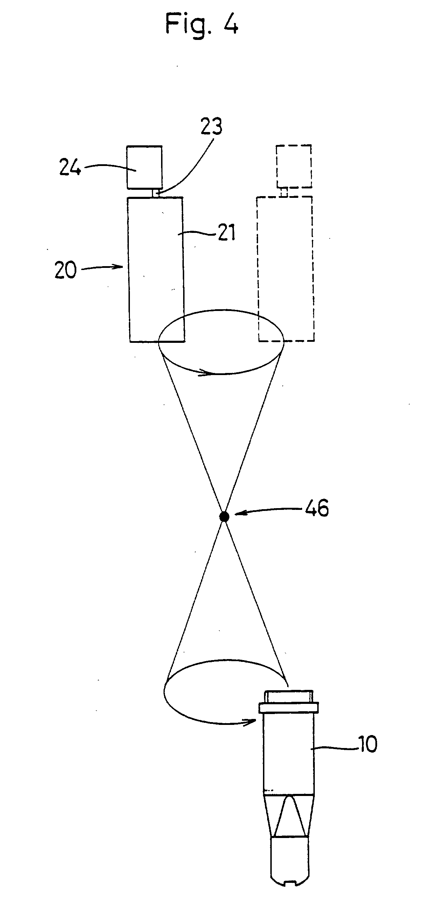

Fig. 1 to Fig. 4 are views showing a mixing apparatus D according to an embodiment of the present invention. Fig. 1 is a side view of the mixing apparatus D. Fig. 2 is a plan view of the mixing apparatus D. Fig. 3 is an enlarged side sectional view of a part (around a linking member) of the mixing apparatus D. Fig. 4 is an explanatory view for explaining the mixing operation of the mixing apparatus D.

In Figs. 1 and 2, the reference numeral 10 represents a vessel made of plastic for containing and holding a subject liquid sample and a liquid reagent and for generating a prescribed reaction by mixing of the sample with the reagent. The vessel 10 is transparent if the change in optical characteristics (scattered light intensity) of the mixed solution is to be measured.

The vessel 10 is gripped and held from the right and left sides by a hand member 12. The hand member 12 comprises a pair of horizontally disposed right and left board-like members and a coil spring 14 stretched over the base end portions of the board-like members. The hand member 12 is constructed in such a manner that the pair of board-like members are pivoted at each of the base end portions to be horizontally revolvable by an urging power of the coil spring 14 at the bottom of a vertically disposed rectangular board-like support member 16.

The reference numeral 20 represents a vibration motor, which is integrally mounted to the support member 16 by upper and lower board-like mounting members 18 and 22 at the top of the support member 16. The vibration motor 20 comprises a DC coreless motor body 21 and an eccentric weight 24, the weight 24 being eccentrically mounted to a motor shaft 23 of the motor body 21. The rotation of the weight 24 allows the motor body 21 to conically revolve and vibrate. In order to increase the amplitude of the conical revolution vibration, the weight 24 preferably has a large moment with respect to the motor shaft 23. In view of this, the weight 24 is adapted to have a semicircular cross section (the shape of a circular cylinder longitudinally halved along the motor shaft 23).

The reference numeral 26 represents a mobile member which moves in a prescribed region by a mechanism not shown. The mobile member 26 moves in three-dimensional directions (in X, Y, and Z axis directions). The mechanism therefor can be provided by a known art.

The mobile member 26 and the support member 16 are linked by a linking member 28. However, they are not completely fixed but are linked in a semi-fixed state. The semi-fixed state herein referred to represents a state in which the support member 16 is linked to the mobile member 26 with some three-dimensional degree of freedom.

Referring to Fig. 3, the periphery (the linking portion) of the linking member 28 will be hereinafter explained. The mobile member 26 and the support member 16 are linked by screwing the linking member 28 with nut members 30 and 32. The reference numeral 44 represents a protection cover. The linking member 28 comprises a resilient member 42, via which the mobile member 26 is linked to the support member 16. More specifically explained, the linking member 28 is constructed by contacting and linking a first member 34 and a second member 36 with each other by using the resilient member 42, the first and second members 34 and 36 being made of metal and having flange portions 38 and 40, respectively.

In other words, the linking is such that the mobile member 26 contacts with the flange portion 38 on one side, the support member 16 contacts with the flange portion 40 on the other side, and there is a gap between the mobile member 26 and the support member 16.

Here, the resilient member 42 has a disk-like shape with a diameter of 7mm and a thickness of 1mm and, specifically, is made of an ether type polyurethane ("Sorbothane" manufactured by Sanshin Enterprises Co., Ltd. in Japan) having a hardness in the range of 30 to 50 when measured by a Shore (OO scale) hardness meter.

The linking member 28 has only to link the support member 16 to the mobile member 26 so that the support member 16 is conically revolvable with the linking portion serving as a fulcrum. The same operational effect can be obtained by providing a male screw member and an O-ring and by linking the members 26 and 16 using a nut member via the O-ring.

The support member 16 has only to be loosely linked to the mobile member 26. The linking between the support member 16 and the mobile member 26 may be constructed as follows in order to generate the conical revolution movement. The linking member 28 is formed with a bar-like member having a screw portion and with a resilient member (for example, an O-ring or a coil spring) penetrating through the bar-like member. The mobile member 26 and the support member 16 respectively contact with the resilient member, and there is a gap between the mobile member 26 and the support member 16. Alternatively, the linking member 28 may be constructed by providing a ball member between the first and second members.

By thus linking the mobile member 26 to the support member 16, the support member 16 revolves conically with the linking portion serving as a fulcrum 46 by the rotation of the vibration motor 20, as shown in Fig. 4. In accordance with this movement, the liquid (sample + reagent) contained in the vessel 10 conically revolves to be suitably mixed.

Figs. 1 to 4 show a construction in which, in view of facility in construction and others, the vibration motor 20 is disposed on one side of the support member 16, the hand member 12 is disposed on the other side, and the linking member 28 (the fulcrum 46) is disposed therebetween. Alternatively, however, the vibration motor 20 may be provided on the side of the hand member 12.

Next, an overall operation of the mixing apparatus D will be briefly explained. First, by the forward movement of the mobile member 26, the hand member 12 proceeds toward an empty vessel 10 to grip and hold the empty vessel 10 at a predetermined position by pressing.

Then, the hand member 12 rises and moves to a different position so that a sample of 50 to 100 µl is dispensed into the vessel 10. The hand member 12 further moves to a different position so that a reagent of 50 to 200 µl is dispensed into the vessel 10.

After the reagent is dispensed, the liquid is immediately mixed for a prescribed amount of time (for example, 0.6 seconds) by the driven motion of the vibration motor 20. The rotation speed of the vibration motor 20 for mixing may be any amount of time, for example, 7500 ± 500 rpm. After mixing, the hand member 12 moves to a measuring portion to release the vessel 10, whereby the optical characteristics of the mixed liquid in the vessel 10 are measured. After the measurement, the vessel 10 is discarded.

The mixing apparatus D is constructed in such a manner that the vibration motor 20 is disposed on one side of the support member 16, the hand member 12 is disposed on the other side, and the linking member 28 (the fulcrum 46) is disposed therebetween, whereby the support member 16 conically revolves by the driven motion of the vibration motor 20 with the linking portion serving as the fulcrum 46. Also, the vibration motor 20 comprises the eccentric weight 24, and the linking member 28 comprises the resilient member 42. Accordingly, the liquid (sample + reagent) in the vessel 10 conically revolves to be suitably mixed. Further, when the vibration motor 20 stops, the support member 16 halts at its original location, irrespective of the position at which the motor shaft 23 stops.

Since the mixing apparatus according to the present invention is constructed in such a manner as in the above, the mixing apparatus produces the following remarkable effects.

In other words, the mixing apparatus according to the present invention comprises a hand member for holding a vessel for containing a liquid to be mixed, a support member for supporting the hand member, a mobile member which moves in a predetermined region, a vibration motor mounted to the support member, and a linking member for linking the support member to the mobile member so that the support member is conically revolvable with respect to the mobile member when the vibration motor is driven. Accordingly, the eccentric rotational movement of the vibration motor mounted to the support member is converted to the conical revolving movement by the linking member, thereby allowing the hand member to revolve. By this movement, the liquid in the vessel held by the hand member revolves along the inner surface of the vessel to be suitably mixed. This can prevent the possibility of mixing nonuniformity and contamination. Also, the mixing apparatus according to the present invention employs a small and simple mechanism, and is advantageous with respect to costs. Moreover, the mixing apparatus does not particularly need a control mechanism for determining the position of the vessel.

In the mixing apparatus according to the invention, the hand member is disposed on one side of the support member, and the vibration motor is disposed on the other side of the support member, thereby allowing the support member to revolve with a fulcrum at a linking portion linking the support member to the mobile member. Therefore, the effect produced by the mixing apparatus according to the present invention can be simply and securely achieved by a mixing apparatus which employs a small and simple mechanism and which is advantageous with respect to costs.

In the mixing apparatus according to the invention, the linking member comprises a resilient member via which the support member is linked to the mobile member, allowing the support member to conically revolve well in such a simple construction. Therefore, the effect produced by the mixing apparatus according to the present invention can be more securely achieved.

In the mixing apparatus according to the invention, the vibration motor comprises a motor body and a weight, the weight being eccentrically mounted to a motor shaft of the motor body and having a semicircular cross section. Therefore, the effect produced by the mixing apparatus according to the present invention can be more effectively and smoothly obtained.

## Claims

1. A mixing apparatus comprising :
a hand member (12) for holding a vessel (10) for containing a liquide to be mixed ;
a support member (16) for supporting the hand member (12) ;
a mobile member (26) which moves in a predetermined region ;
a vibration motor (20) mounted to the support member (16) ; and
a linking member (28) for linking the support member (16) to the mobile member (26) so that the support member (16) is capable of conical revolution movement with respect to the mobile member (26) when the vibration motor (20) is driven, thereby enabling conical revolution movement of the vessel (10) held by the hand member (12).

2. A mixing apparatus according to claim 1, in which the hand member (12) is disposed on one side of the support member (16), and the vibration motor (20) is disposed on the other side of the support member (16), thereby allowing the support member (16) to perform the conical revolution movement with a fulcrum (46) at a linking portion linking the support member (16) to the mobile member (26).

3. A mixing apparatus according to claim 1, in which the linking member (28) comprises a resilient member (42) via which the support member (16) is linked to the mobile member (26).

4. A mixing apparatus according to claim 1, in which the hand member (12) comprises a pair of horizontally disposed board-like members and a coil spring (14) stretched over the end portions of the board-like members so that the pair of the board-like members are pivoted at each of the base end portions to be horizontally rotatable by an urging power of the coil spring (14).

5. A mixing apparatus according to claim 1, in which the support member (16) has a vertically disposed rectangular board-like shape.

6. A mixing apparatus according to claim 1, in which the mobile member (26) is capable of moving in three-dimensional directions.

7. A mixing apparatus according to claim 1, in which the vibration motor (20) comprises a motor body (21) and a weight (24), the weight (24) being eccentrically mounted to a motor shaft (23) of the motor body and having a semicircular cross section.

8. A mixing apparatus according to claim 1, in which the linking member (28) is constructed by contacting and linking a first member and a second member with each other by intermediation of a resilient member (42) made of polyurethane, the first member (34) being made of a metal and having a flange portion with which the mobile member contacts, and the second member (36) being made of a metal and having a flange portion (38, 40) with which the support member contacts.

## Patentansprüche

1. Mischgerät mit:
einem Griffelement (12) zum Halten eines Behälters (10) zum Aufnehmen einer zu mischenden Flüssigkeit;
einem Halteelement (16) zum Halten des Griffelements (12) ;
einem beweglichen Element (26), welches sich in einem vorbestimmten Bereich bewegt;
einem Vibrationsmotor (20), welcher an dem Halteelement (16) angebracht ist; und
einem Verbindungselement (28) zum Verbinden des Halteelements (16) mit dem beweglichen Element (26), so dass das Halteelement (16) eine konische Drehbewegung bezüglich des beweglichen Elements (26) ausführen kann, wenn der Vibrationsmotor (20) angetrieben wird, wodurch eine konische Drehbewegung des Behälters (10) möglich wird, welcher durch das Griffelement (12) gehalten wird.

2. Mischgerät nach Anspruch 1, wobei das Griffelement (12) an einer Seite des Halteelements (16) und der Vibrationsmotor (20) an der anderen Seite des Halteelements (16) vorgesehen ist, wodurch das Halteelement (16) die konische Drehbewegung mit einem Drehpunkt (26) in einem Verbindungsbereich durchführen kann, welcher das Halteelement (16) mit dem beweglichen Element (26) verbindet.

3. Mischgerät nach Anspruch 1, wobei das Verbindungselement (28) ein nachgiebiges Element (42) aufweist, mittels dessen das Halteelement (16) mit dem beweglichen Element (26) verbunden ist.

4. Mischgerät nach Anspruch 1, wobei das Griffelement (12) ein Paar von horizontal angeordneten plattenartigen Elementen sowie eine Schraubenfeder (14) aufweist, welche über die Endbereiche der plattenartigen Elemente hinüber gestreckt ist, so dass das Paar von plattenartigen Elementen an den Basisendbereichen gelenkig gelagert sind, um horizontal drehbar zu sein durch eine Kraft der Schraubenfeder (14).

5. Mischgerät nach Anspruch 1, wobei das Halteelement (16) eine vertikal angeordnete rechteckige plattenartige Gestalt hat.

6. Mischgerät nach Anspruch 1, wobei das bewegliche Element (26) sich dreidimensional bewegen kann.

7. Mischgerät nach Anspruch 1, wobei der Vibrationsmotor (20) einen Motorkörper (21) und ein Gewicht (24) aufweist, wobei das Gewicht (24) exzentrisch an einer Motorwelle (23) des Motorkörpers angebracht ist und einen halbkreisförmigen Querschnitt hat.

8. Mischgerät nach Anspruch 1, wobei das Verbindungselement 28 durch Kontaktieren und Verbinden eines ersten mit einem zweiten Element durch Zwischenwirkung eines nachgiebigen Elements (42) aus Polyurethan aufgebaut ist, wobei das erste Element (34) aus Metall besteht und einen Flanschbereich aufweist, mit welchem das bewegliche Element in Kontakt steht, und wobei das zweite Element (36) aus Metall besteht und einen Flanschbereich (38, 40) aufweist, mit welchem das Halteelement in Kontakt steht.

## Revendications

1. Appareil mélangeur comprenant :
un élément formant poignée (12) pour tenir un récipient (10) destiné à contenir un liquide à mélanger;
un élément de support (16) servant à supporter l'élément formant poignée (12);
un élément mobile (26) qui se déplace dans une zone prédéterminée;
un moteur (20) de production de vibrations, monté de manière à supporter l'élément (16); et
un élément de liaison (28) servant à relier l'élément de support (16) à l'élément mobile (26) de telle sorte que l'élément de support (16) peut exécuter un mouvement de révolution conique par rapport à l'élément mobile (26) lorsque le moteur (20) fonctionne, ce qui permet d'obtenir un mouvement de révolution conique du récipient (10) tenu au moyen de l'élément formant poignée (12).

2. Appareil de mélange selon la revendication 1, dans lequel l'élément formant poignée (12) est disposé d'un côté de l'élément de support (16), et le moteur (20) de production de vibrations est disposé de l'autre côté de l'élément de support (16), ce qui permet à l'élément de support (16) d'effectuer un mouvement de révolution conique avec un pivot (46) situé au niveau de la partie de liaison qui relie l'élément de support (16) à l'élément mobile (26).

3. Appareil de mélange selon la revendication 1, dans lequel l'organe de liaison (28) comprend un élément élastique (42), au moyen duquel l'élément de support (16) est relié à l'élément mobile (26).

4. Dispositif de mélange selon la revendication 1, dans lequel l'élément formant poignée (12) comprend un élément en forme de panneau disposé horizontalement et un ressort hélicoïdal (14) est tendu sur les parties d'extrémité des éléments en forme de panneaux de sorte que le couple des éléments en forme de panneaux peut pivoter sur chacune des parties d'extrémité de base devant pivoter horizontalement sous l'action d'une puissance de sollicitation du ressort hélicoïdal (14).

5. Appareil de mélange selon la revendication 1, dans lequel l'élément de support (7) possède une forme de panneau rectangulaire disposé verticalement.

6. Appareil de mélange selon la revendication 1, dans lequel l'élément mobile (26) peut se déplacer dans des directions suivant trois dimensions.

7. Appareil de mélange selon la revendication 1, dans lequel le moteur (20) de production de vibrations comprend un corps de moteur (21) et un poids (24), le poids (24) étant monté de façon excentrique sur un arbre (23) du corps du moteur et possédant une section transversale semi-circulaire.

8. Appareil de mélange selon la revendication 1, dans lequel un élément d'articulation (28) est constitué par mise en contact et réunion d'un premier élément et d'un second élément entre eux par l'intermédiaire d'un élément élastique (42) formé de polyuréthane, le premier élément (34) étant formé d'un métal et comportant une partie formant bride, avec laquelle l'élément mobile est en contact, et le second élément (36) étant formé d'un métal et possédant une partie de bride (38,40), avec laquelle l'élément de support est en contact.
